# EUROPEAN PATENT APPLICATION

(11) **EP 3 900 619 A1**
(43) Date of publication of application: **27.10.2021**
(21) Application number: 19898956.8
(22) Date of filing: 10.09.2019
(51) Int. Cl.: A61B 5/0205, A61B 5/1455

(54) **WEARABLE APPARATUS, AND ACCESSORY FOR TERMINAL DEVICE**

(30) Priority: 18.12.2018 CN 201822151091 U
(71) Applicant: Anhui Huami Information Technology Co., Ltd., Hefei, Anhui 230088 (CN)
(72) Inventor: WANG, Bing, Hefei, Anhui 230088 (CN); ZHAO, Yajun, Hefei, Anhui 230088 (CN)
(74) Representative: Angerhausen, Christoph
(86) International application number: PCT/CN2019/105202
(87) International publication number: WO 2020/125082

(57) **Abstract**

A wearable apparatus (100) and an accessory for a terminal device are provided. The wearable apparatus (100) includes a body (1), a heart rate acquisition device (2), and a blood oxygen saturation acquisition device (3). The body (1) includes a ring (12) provided with a wearing space (121). The heart rate acquisition device (2) is mounted on the body (1) and configured to acquire heart rate data. The blood oxygen saturation acquisition device (3) is mounted on the body (1) and configured to acquire blood oxygen saturation data.

## Description

### CROSS-REFERENCE TO RELATED APPLICATION(S)

The present application is based on and claims priority to a Chinese Patent Application No. 201822151091.5 filed on December 18, 2018, the contents of which are hereby incorporated by reference in their entireties.

### TECHNICAL FIELD

The present application relates to the field of healthcare monitoring, and in particular, to a wearable apparatus and an accessory for a terminal device.

### BACKGROUND

With the improvement of people's living standards, more and more attention has been paid to health. There are already different types of products for detecting heart rate and blood oxygen saturation on the market, but these products are often inconvenient to carry and unable to measure heart rate and blood oxygen saturation and other physical sign information in real time. For example, especially during rest, using these products for measurements will often affect a user's sleep. On the other hand, hypertension, heart disease, stroke, diabetes, and other diseases may lead to sleep apnea, which makes detection of physical signs during sleep particularly important.

### SUMMARY

The present disclosure provides a wearable apparatus and an accessory for a terminal device that can detect physical sign information in real time.

The present disclosure provides a wearable apparatus, including a body, a heart rate acquisition device, and a blood oxygen saturation acquisition device. The body includes a ring provided with a wearing space. The heart rate acquisition device is mounted on the body and configured to acquire heart rate data. The blood oxygen saturation acquisition device is mounted on the body and configured to acquire blood oxygen saturation data.

Further, the body includes a base connected to the ring, the heart rate acquisition device includes a light source and a photoelectric collector that are mounted on the base.

Further, the body includes a base connected to the ring, the heart rate acquisition device includes a light source mounted on the base and a photoelectric collector mounted on the ring.

Further, the light source is mounted on an inner surface of the ring and faces the photoelectric collector.

Further, the wearable apparatus includes at least two light sources, and the light sources are arranged in a circumferential direction of the ring.

Further, the blood oxygen saturation acquisition device includes a dual-wavelength emitting tube.

Further, the body includes a base, and the ring is rotatably connected to the base.

Further, the wearable apparatus includes at least one of a wireless transmission module or a wired transmission module.

Further, the wearable apparatus includes a connecting portion, and the wearable apparatus is connected to an external device through the connecting portion.

The present application also provides an accessory for a terminal device, including a protective shell and the wearable apparatus as described above. The protective shell is configured to accommodate the terminal device, and the wearable apparatus is connected to the protective shell.

In the present application, a user can put the wearable apparatus on a finger, which does not affect the user's normal activities or sleep when worn. Physical sign information of the user is acquired in real time through the heart rate acquisition device and the blood oxygen saturation acquisition device, so as to obtain an accurate and reliable detection result.

### BRIEF DESCRIPTION OF DRAWINGS

FIG. 1 is a schematic diagram of an implementation of a wearable apparatus according to the present application;
FIG. 2 is a schematic side view of the wearable apparatus shown in FIG. 1;
FIG. 3 is a schematic diagram of an implementation of a wearable apparatus of the present application; and

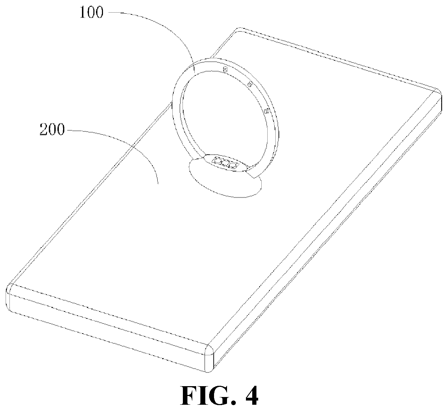

FIG. 4 is a schematic diagram of an implementation of an accessory for a terminal device according to the present application.

### DETAILED DESCRIPTION

Example implementations will be described in detail here, and examples thereof are shown in the accompanying drawings. When the following description refers to the accompanying drawings, unless otherwise indicated, the same numerals in different drawings indicate the same or similar elements. Implementations described in the following example implementations do not represent all implementations consistent with the present application. On the contrary, they are merely examples of apparatus consistent with some aspects of the present application as detailed in the appended claims.

The terms used in the present application are only for the purpose of describing specific implementations, and are not intended to limit the present application. Unless otherwise defined, the technical or scientific terms used in the present application shall have usual meanings understood by those of ordinary skills in the art to which the present disclosure belongs. The "first," "second," and similar words used in the specification and claims of the present application do not denote any order, quantity, or importance, but are only configured to distinguish different components. Similarly, similar words such as "one" or "a" do not mean a quantity limit, but mean that there is at least one. "A plurality of" or "several" means two or more. Similar words such as "including" or "containing" mean that the elements or articles before "including" or "containing" cover the elements or articles listed after "including" or "containing" and their equivalents, and do not exclude other elements or articles. Similar words such as "connect" or "connected" are not limited to physical or mechanical connections, and may include electrical connections, whether direct or indirect. The singular forms of "a," "said," and "the" used in the specification and appended claims of the present application are also intended to include plural forms, unless the context clearly indicates other meanings. It should also be understood that the term "and/or" as used herein refers to and includes any or all possible combinations of one or more associated listed items.

The present implementation provides a wearable apparatus, including a body, a heart rate acquisition device, and a blood oxygen saturation acquisition device. The body includes a ring provided with a wearing space for accommodating a finger. The heart rate acquisition device is mounted on the body and configured to acquire heart rate data. The blood oxygen saturation acquisition device is mounted on the body and configured to acquire blood oxygen saturation data.

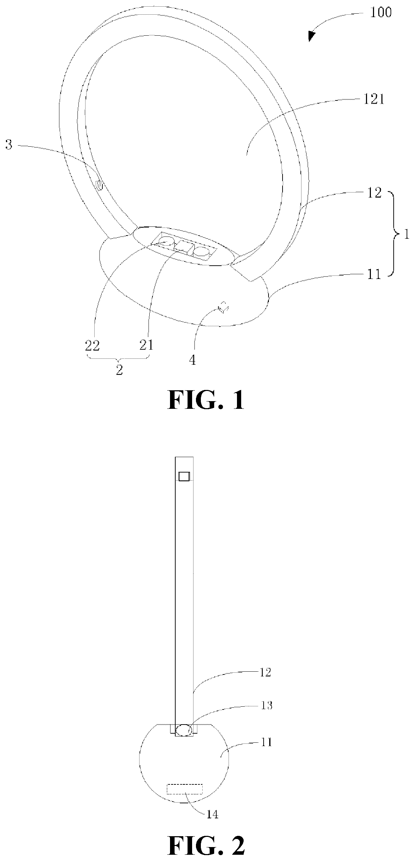

Referring to FIG. 1, wearable apparatus 100 includes body 1, heart rate acquisition device 2, blood oxygen saturation acquisition device 3, and transmission module 4 (indicated by dashed lines as it is invisible) that are mounted on the body. Transmission module 4 is mounted in a recess of the body (base 11), and electrically connected to heart rate acquisition device 2 and blood oxygen saturation acquisition device 3, respectively, and configured to transmit the detected physical sign information to a terminal device and/or a server and/or cloud. In some implementations of the present application, transmission module 4 may be a wired transmission module, such as one having a connection line provided between the wearable apparatus 100 and the terminal device, or may be a wireless transmission module such as a Bluetooth transmission module.

Referring to FIG. 2, body 1 includes base 11 and ring 12 connected to the base. Ring 12 includes wearing space 121 for accommodating a finger of a user. In one implementation, ring 12 is rotatably connected to base 11. In some implementations of the present application, ring 12 is rotatably connected to base 11 via shaft 13.

In this implementation, heart rate acquisition device 2 is a photoplethysmography sensor, which includes light source 21 and photoelectric collector 22. Light source 21 is configured to emit detection light (for example, green light), and light source 21 and photoelectric collector 22 are both mounted on base 12. In this implementation, light source 21 and photoelectric collector 22 are integrated on a circuit board, that is, the photoplethysmography sensor in this implementation is a reflection-type photoplethysmography sensor. To increase comfort, base 11 is provided with a cavity structure, and heart rate acquisition device 2 is accommodated in the cavity structure.

When the detection light emitted by light source 21 irradiates the skin surface of the finger of the user, since light absorption coefficients of light absorption substances (such as hemoglobin of the subcutaneous tissue of the human body) and blood concentrations remain unchanged in the entire blood circulation system, an optical path of reflected light is periodically changed with the heartbeat. In the diastolic period, the peripheral blood volume is smaller, the optical path is the shorter, the light absorption is also smaller, and the detected light intensity is smaller; while in the systolic period it is just the opposite, and the detected light intensity is correspondingly larger. In this way, real-time heart rate data of the user can be obtained through heart rate acquisition device 2.

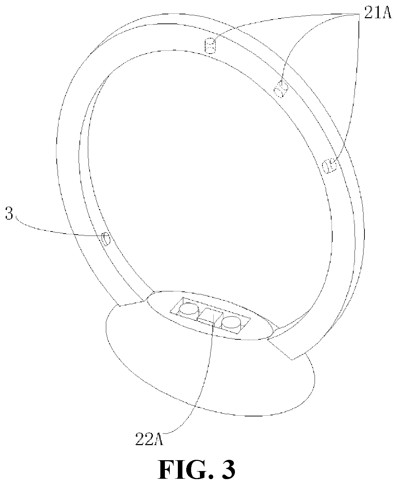

Referring to FIG. 3, in another implementation, the heart rate acquisition device is a refraction-type photoplethysmography sensor, that is, the detected light is refracted by the finger and then absorbed by the photoelectric collector. The heart rate acquisition device includes light source 21A and photoelectric collector 22A. Light source 21A is mounted on ring 12, and photoelectric collector 22A is mounted on base 11. In some implementations of the present application, light source 21A is mounted on an inner surface of ring 12 and faces photoelectric collector 22A. In some implementations, there may be one light source 21A. To further improve the detection accuracy, in some other implementations, there may also be two or more light sources that can be arranged in a circumferential direction of ring 12. In this implementation in FIG. 3, the number of light sources 21A is three. The refraction-type photoplethysmography sensor has higher detection accuracy than the reflection-type photoplethysmography sensor.

In this implementation, blood oxygen saturation acquisition device 3 includes a dual-wavelength light-emitting tube, which emits light of two different wavelengths. It should be noted that a light-emitting tube capable of emitting a plurality of (three or more) wavelengths can also achieve the purpose of a dual-wavelength light-emitting tube in the present application. The replacement of the dual-wavelength light-emitting tube described in the present application with the light-emitting tube that can emit a plurality of (three or more) wavelengths should also fall within the protection scope of the present application.

For example, the dual-wavelength light-emitting tube can emit, e.g., 660 nm red light and 910 nm infrared light. When the hemoglobin does not carry oxygen molecules, reduced hemoglobin absorbs the 660 nm red light relatively strongly, but absorbs the 910 nm infrared light relatively weakly. When the hemoglobin carries red blood cells with oxygen molecules, oxygenated hemoglobin absorbs the 660 nm red light relatively weakly, but absorbs the 910 nm infrared light relatively strongly. Therefore, in blood oxygen measurement, a difference between the light absorption of different wavelengths by the reduced hemoglobin and the oxygenated hemoglobin is detected, and the measured data difference is the basis data for measuring the blood oxygen saturation. Specifically, the dual-wavelength light-emitting tube (i.e., blood oxygen saturation acquisition device 3) is controlled to alternately emit light and cooperates with an analog front end to acquire data, and accurate blood oxygen saturation data can be obtained through digital circuit processing.

Base 11 of the ring is also provided with a prompting device (not shown) configured for event reminding. An event can include, for example, completion of heart rate detection, completion of blood oxygen saturation detection, alarm clock, apnea during night sleep, or the like. In this implementation, the prompting device may be a micro vibration motor. In other implementations, the prompting device may also emit other optical or acoustic prompts.

In one implementation, wearable apparatus 100 further includes connecting portion 14 configured for connecting with an external device. Therefore, wearable apparatus 100 is connected to a terminal device and/or an accessory for the terminal device. The terminal device may be a mobile phone, a tablet computer, a smart watch, a VR device, an AR device, and the like. The "connection" here may be a direct connection, or an indirect connection to the terminal device and/or the accessory for the terminal device through an intermediate connector, and it may be a detachable connection or a non-detachable connection. It can be understood that when the terminal device or the accessory for the terminal device is relatively large, the detachable connection can be selected. When the terminal device or the accessory for the terminal device is relatively small, it is more convenient to carry and wear at this time to meet the needs of real-time measurement. Therefore, even if the non-detachable connection is selected, the objective of the present disclosure can still be realized.

Connecting portion 14 may be, for example, a magnet, which is accommodated in base 11 and can be detachably connected to the terminal device through the magnetic force. The terminal device or the accessory for the terminal device may be provided with components made of magnet or magnetic material. In other implementations, the connecting portion may also be a buckling structure, in which case the connecting portion protrudes from base 11. The connecting portion may be provided on base 11 or it can be provided on ring 12.

The present application also provides an accessory for a terminal device. The terminal device is, for example, a mobile phone. The accessory for a terminal device includes protective shell 200 and wearable apparatus 100 in any of the foregoing implementations. Wearable apparatus 100 is connected to protective shell 200. The protective shell is configured to accommodate the terminal device and can protect the terminal device from being impacted by an external force. In some implementations of the present application, wearable apparatus 100 is detachably connected to protective shell 200, for example, by a magnetic force. Connection may also be achieved by buckling, locking, or the like. In an implementation, protective shell 200 may be provided with a thin iron sheet (which is not shown, and may also be an element made of another magnetic material or a magnet) to be detachably connected to the magnet provided on wearable apparatus 100.

The accessory for the terminal device may include the wearable apparatus and the protective shell, or may only include the protective shell.

Connecting wearable apparatus 100 to protective shell 200 can increase the grip of a mobile phone, and the protective shell can also be used as a stand. After wearable apparatus 100 is disconnected, it can be sleeved on a finger to detect the heart rate and blood oxygen saturation during sleep throughout the night. By judging a change in the blood oxygen saturation during sleep and detecting the degree of impact of apnea on sleep, a reliable report for sleep quality of the whole night can be provided. The obtained physical sign information can be transmitted to the terminal device through the transmission module, and data can be previewed, stored, processed, and uploaded to the cloud and/or a medical facility through an application of a smart phone, so as to conduct a real-time health assessment of the user and provide appropriate advices.

In the present application, the wearable apparatus can be sleeved on a finger and does not affect a user's normal activities or sleep when worn. Physical sign information of the user is acquired through the heart rate acquisition device and the blood oxygen saturation acquisition device, so as to obtain an accurate and reliable detection result in real time.

The above description is only preferred implementations of the present application and is not intended to limit the present application. Any modification, equivalent replacement, improvement, and the like made within the spirit and principle of the present application shall be included in the protection scope of the present application.

## Claims

1. A wearable apparatus, comprising:
a body comprising a ring provided with a wearing space;
a heart rate acquisition device mounted on the body and configured to acquire heart rate data; and
a blood oxygen saturation acquisition device mounted on the body and configured to acquire blood oxygen saturation data.

2. The wearable apparatus according to claim 1, wherein the body comprises a base connected to the ring, and the heart rate acquisition device comprises a light source and a photoelectric collector both mounted on the base.

3. The wearable apparatus according to claim 1 or 2, wherein the body comprises a base connected to the ring, and the heart rate acquisition device comprises a light source mounted on the ring and a photoelectric collector mounted on the base.

4. The wearable apparatus according to claim 3, wherein the light source is mounted on an inner surface of the ring and faces the photoelectric collector.

5. The wearable apparatus according to claim 4, wherein the wearable apparatus comprises at least two light sources, and the light sources are arranged in a circumferential direction of the ring.

6. The wearable apparatus according to any one of claims 1 to 5, wherein the blood oxygen saturation acquisition device comprises a dual-wavelength emitting tube.

7. The wearable apparatus according to any one of claims 1 to 6, wherein the body comprises a base, and the ring is rotatably connected to the base.

8. The wearable apparatus according to any one of claims 1 to 7, wherein the wearable apparatus comprises at least one of a wireless transmission module or a wired transmission module.

9. The wearable apparatus according to any one of claims 1 to 8, wherein the wearable apparatus comprises a connecting portion, and the wearable apparatus is connected to an external device through the connecting portion.

10. A terminal device accessory, comprising:
a protective shell configured to accommodate a terminal device; and
a wearable apparatus according to any one of claims 1 to 9, wherein the wearable apparatus is connected to the protective shell.
